# EUROPEAN PATENT APPLICATION

(11) **EP 3 056 243 A1**
(43) Date of publication of application: **17.08.2016**
(21) Application number: 14852262.6
(22) Date of filing: 07.10.2014
(51) Int. Cl.: A61N 1/30, A61K 9/70, A61M 37/00

(54) **PORTABLE IONTOPHORETIC SYSTEM USING SHEET MASK**

(30) Priority: 07.10.2013 KR 20130118899
(71) Applicant: Yonwoo Co., Ltd., Incheon 404-250 (KR)
(72) Inventor: JUNG, Hyo-Sun, Incheon 404-250 (KR)
(74) Representative: Nordic Patent Service A/S
(86) International application number: PCT/KR2014/009428
(87) International publication number: WO 2015/053532

(57) **Abstract**

The present invention relates to a portable iontophoretic system and sheet mask, particularly a portable iontophoretic system which has a current-carrying part for a metal contact formed on a side surface of a portable iontophoresor and enables iontophoresis to be carried out on the skin when a user holds the current-carrying part in a state of the disposable sheet mask applied on a user's skin by connecting the disposable sheet mask to the iontophoresor with a tong, thereby providing an efficient skin care effect through a simple structure.

## Description

### BACKGROUND OF THE INVENTION

The present invention relates to a portable iontophoretic system and sheet mask, particularly a portable iontophoretic system which has a current-carrying part for a metal contact formed on a side surface of a portable iontophoresor and enables iontophoresis to be carried out on the skin when a user holds the current-carrying part in a state of the disposable sheet mask applied on a user's skin by connecting the disposable sheet mask to the iontophoresor with a tong, thereby providing an efficient skin care effect through a simple structure.

Generally, for a facial skin care, a user has been utilizing a iontophoresor for interfusing cosmetics or skin care products deep into the skin after applying cosmetics or skin care products. The iontophoresor radiates tiny electric currents and interfuses cosmetics or skin care products deep into the skin by electric repulsive power of electric charge.

A "Cosmetic Case Having Ion Injector" as the above is disclosed in Korean Patent No. 10-0900788 (hereinafter as "the registered patent"). The said registered patent has a cosmetic storage container provided in the interior of a lower case and with a current-carrying part provided at an upper case, which enables introduction of ions onto a user's skin with discharge of cosmetics, thereby providing efficient skin care.

However, there are some problems wherein efficient skin care through introduction of ions can be achieved only when a user herself should manipulate the current-carrying part in state that a current-carrying part is contacted to skin, and a user herself should hold a gauge being used while cleansing or whitening, thereby resulting in user inconvenience.

### SUMMARY OF THE INVENTION

The present invention is devised to solve the said problems above, and its goal is to have a current-carrying part for a metal contact formed on a side surface of a portable iontophoresor and enable iontophoresis to be carried out on the skin when a user holds the current-carrying part in a state where the disposable sheet mask is applied on the face of the user by connecting the disposable sheet mask to the iontophoresor with tongs, thereby providing an efficient skin care effect through a simple structure.

To solve the said problems above, a portable iontophoretic system and sheet mask to be contacted to a user's face comprises a sheet mask with cosmetic fluid applied; a iontophoresor that has a battery providing electric energy and is configured to have one of two electrodes of the above battery electrically connected with a user's skin; and a connection line having one end connected with the sheet mask while the other end is connected with the rest of the two electrodes provided in the iontophoresor and delivering micro-electrical currents of the said battery.

Further, both sides of the said iontophoresor comprise a current-carrying part for sending electric currents through metal contact with the user's skin.

Further, the said iontophoresor, in addition, comprises button parts controlling an on/off control and a dynamics of electrical currents.

As mentioned above, the present invention has a configuration wherein a current-carrying part for a metal contact is formed on a side surface of a portable iontophoresor and iontophoresis is carried out on the skin when a user holds the current-carrying part in a state of the disposable sheet mask applied on the face of the user by connecting the disposable sheet mask to the iontophoresor with tongs, and thereby provides an efficient skin care effect through a simple structure.

### BRIEF DESCRIPTION OF THE DRAWINGS

Fig. 1 is an exploded view illustrating a configuration of an iontophoresor and a connection line of a portable iontophoretic system and sheet mask according to an exemplary embodiment of the present invention.
Fig. 2 is an assembled view illustrating a configuration of *a* portable iontophoretic system and sheet mask according to an exemplary embodiment of the present invention.
Figs. 3 to 5 are explanatory drawings illustrating an operational method of *a* portable iontophoretic system and sheet mask according to an exemplary embodiment of the present invention.

### DETAILED DESCRIPTION OF PREFERRED EMBODIMENTS

Hereinafter, exemplary embodiments of the present invention will be described in detail with reference to the accompanying drawings. The same reference numerals provided in the drawings indicate the same members.

Fig. 1 is an exploded view illustrating a configuration of a iontophoresor and a connection line of a portable iontophoretic system using a sheet mask according to an exemplary embodiment of the present invention. Fig. 2 is an assembled view illustrating a configuration of *a* portable iontophoretic system using a sheet mask according to an exemplary embodiment of the present invention. Figs. 3 to 5 are explanatory drawings illustrating an operational method of *a* portable iontophoretic system using a sheet mask according to an exemplary embodiment of the present invention.

Referring to Figs. 1 to 5, a portable iontophoretic system using a sheet mask according to an exemplary embodiment of the present invention includes a sheet mask 100, a iontophoresor 200, and a connection line 300.

The sheet mask 100, comprised of a packing sheet containing a raw cosmetic material for facial and skin care and moisture for hydration, a felt sheet, and a parting paper, can achieve an efficient skin care through giving moisture and nutrients to the face and skin when it is attached on the face for a while and then detached. The sheet mask 100 applied with cosmetic fluid that is contacted to a user's face is for a single use. In the present invention, the sheet mask 100 is contacted to a user's skin with the fluid content such as cosmetic fluid, and thereby has a feature of acting as medium to deliver ions onto the user's skin when a iontophoresor 200 is operated.

The iontophoresor 200 causes cosmetics or skincare supplements to infiltrate deep into the skin by repelling power of electric charge by radiating micro electric currents and having a battery 240 supplying electric energy to the inside thereof. Either of two electrodes, positive (+) or negative (-), of the battery 240 is configured to be contacted to a user's skin electrically whereas the other electrode is configured to be connected to a connection line that delivers micro-electrical currents to the sheet mask 100.

A pair of insert holes 221 are inserted at a front case 210 of the iontophoresor 200 so as for button parts 212, 213 to be inserted, wherein the button parts 212, 213 connected to an electronic control board 250 control an on/off control and a dynamics of power supply of the iontophoresor 200.

The button parts 213, 213 have a "+" mark on a button part 212 placed at an upper part for user's easy controlling dynamics of electric currents, whereas having a " - " mark on a button part 212 placed at an lower part, and the power supply is turned on when the button part 212 with a " + " mark is pressed for three seconds, whereas the power supply is turned off when the button part 213 with a " - " mark is pressed for three seconds.

Further, it is preferred that a status display part 214 is provided at the front case 210 to be able to display an operation status of the iontophoresor 200 in LED lamp when the button parts 212, 213 are being operated.

Meanwhile, on an upper end of a rear case 220 of the iontophoresor 200 is provided a combining part 221 to which a connection line 300 connected to the sheet mask 100 for delivering electric currents to the sheet mask is connected whereas on the rear surface is provided a battery cover coupling hole 222 to which a battery cover 230 is combined for exchanging a battery 240.

The present invention has a feature in that at both sides of the iontophoresor 200 is provided a current-carrying part 260 for being capable of sending electricity by the contact of a user's skin and a metal. When a user presses the current-carrying part 260 while wearing the sheet mask 100, electric currents flow smoothly, thereby enabling a fluid content that is applied on the sheet mask 100 to infiltrate deep into the user's skin and bringing about efficient skin care.

The connection line 300, one end of which is connected to the sheet mask and the other of which is connected to the remaining electrode not to the electrode for contacting to the user's skin among the two electrodes of the battery 240 provided at the iontophoresor 200, delivers micro electrical currents of the battery 240 to the sheet mask 100, wherein the connection line 300 is combined to a combining part 221 provided at an end of the iontophoresor 200, and it is preferable that a tong 310 is provided at an end of the connection line 300 to be connected to the sheet mask 100.

The connection line 300 delivers electric currents through the tong 310 made of metal and makes it possible to introduce ions into a user's skin.

In the following, referring to Figs. 3 to 5, a portable iontophoretic system using a sheet mask according to an exemplary embodiment of the present invention will be explained. Figs. 3 to 5 are explanatory drawings illustrating an operational way of a portable iontophoretic system using a sheet mask according to an exemplary embodiment of the present invention.

Referring to Figs. 3 to 5, a portable iontophoretic system using a sheet mask according to an exemplary embodiment of the present invention has a sheet mask 100 attached on a user's face by means of a tong 310 connected to the sheet mask 100 at the end of a connection line 300 connected to the iontophoresor 200.

Next, after a user pressurizes the current-carrying part 260 of the iontophoresor 200 with a hand and presses the button part 212 for as long as three seconds, the power of the iontophoresor 200 is on. Then, electric currents supplied from the battery 240 move along the connection line 300 and delivers micro electric currents to the sheet mask 100; as a result, content applied on the sheet mask will be infiltrated deep into the user's skin and bring efficient skin care.

As described above, optimal embodiments have been disclosed in the drawings and the specification. Although specific terms have been used herein, these are only intended to describe the present invention and are not intended to limit the meanings of the terms or to restrict the scope of the present invention as disclosed in the accompanying claims. Accordingly, those skilled in the art will appreciate that various modifications and other equivalent embodiments are possible from the above embodiments. Therefore, the scope of the present invention should be defined by the technical spirit of the accompanying claims.

## Claims

1. A portable iontophoretic system and sheet mask, comprising:
a sheet mask (100) applied with a cosmetic fluid to be attached on a user's face;
a iontophoresor (200) provided with a battery (240) for supplying electric energy, wherein
one of two electrodes of the battery (240) is electrically connected to a user's skin; and
a connection line (300), one side of which is connected to the sheet mask (100) and the other of which is connected to one of two electrodes of a battery (240) provided at the iontophoresor (200), thereby delivering micro electric currents from the battery (240) to the sheet mask.

2. The portable iontophoretic system and sheet mask of claim 1, wherein a current-carrying part (260) is provided at both sides of the iontophoresor (200) as means for sending electricity through a user by metal contact with a user's skin.

3. The portable iontophoretic system and sheet mask of either claim 1 or claim 2, wherein the iontophoresor (200) further comprises button parts (212, 213) as an on/off control of electric power and means for controlling dynamics of electricity.
